# NOUVEAU FASCICULE DE BREVET EUROPEEN

(11) **EP 0 719 540 B2**
(45) Date de publication et mention de la décision concernant l'opposition: **14.11.2001**
(45) Mention de la délivrance du brevet: 21.05.1997
(21) Numéro de dépôt: 95402639.9
(22) Date de dépôt: 22.11.1995
(51) Int. Cl.: A61K 7/48

(54) **Composition cosmétique ou dermatologique sous forme d'une émulsion eau dans huile à teneur élevée en hydroxyacides**
Kosmetische oder dermatologische Wasser-in-Öl Emulsion mit hohem Gehalt an Hydroxysäuren
Cosmetic or dermatological water-in-oil emulsion with high hydroxyacids content

(30) Priorité: 15.12.1994 FR 9415130
(43) Date de publication de la demande: 03.07.1996
(73) Titulaire: CENTRE INTERNATIONAL DE RECHERCHES DERMATOLOGIQUES GALDERMA (C.I.R.D. GALDERMA), 06560 Valbonne (FR)
(72) Inventeur: Willcox, Nathalie, F-06650 Le Rouret (FR); Ferrandis, Agnès, F-06250 Mougins (FR); Preuilh, Isabelle, F-06600 Antibes (FR); Allec, Josiane, 300, Ch. de la Suquette, F-06600 Antibes (FR)
(74) Mandataire: Renard, Emmanuelle

(56) Documents cités:
- EP-A- 0 456 459
- US-A- 4 380 549
- US-A- 4 772 592

## Description

La présente invention concerne une composition cosmétique ou dermatologique du type émulsion eau dans huile comprenant une teneur élevée en hydroxyacides destinée, en application topique, au traitement ou au soin de la peau, des ongles, des cheveux et/ou du cuir chevelu, en particulier pour traiter et/ou prévenir la xérose, l'ichtyose, la kératose actinique et/ou le vieillissement cutané photoinduit.

Il est connu d'utiliser les hydroxyacides pour prévenir ou diminuer les signes dermatologiques du vieillissement de la peau et/ou des cheveux dûs à des facteurs intrinsèques au vieillissement ou encore à des facteurs extérieurs, tels que notamment les rayons UV, la pollution de l'air, le vent, le froid, le chaud, la fumée de cigarettes. Ils sont également connus pour traiter les affections dermatologiques liées à un désordre de la kératinisation de la peau, des ongles et/ou des cheveux, tels que notamment l'acné, la xérose, l'ichtyose et la kératose actinique.

Cependant, ces hydroxyacides sont difficiles à formuler dans une émulsion sous forme de crème ou de lait. En effet, les hydroxyacides, lorsqu'ils sont introduits en grande quantité, rendent la formulation instable et donc difficilement commercialisable.

De plus, ces composés présentent l'inconvénient de provoquer des picotements, des démangeaisons ou des tiraillements après leur application pouvant conduire à un important inconfort. Pour éviter cet inconfort, on peut envisager de libérer de manière lente et différée les hydroxyacides, tout en gardant leur efficacité.

Il est décrit dans le brevet US 4.772.592 une émulsion stable eau dans huile adaptée à une application topique pour traiter l'acné qui comprend un lactate d'alkyle de C1 à C4, une huile siliconée, un émulsifiant liquide non ionique et de l'alcanol de C1 à C4, ces composés étant présents en des quantités spécifiques. Les liquides polaires volatiles, tels que le lactate d'alkyle et l'alcanol, forment des ingrédients essentiels de cette émulsion car ils permettent en association avec l'huile siliconée de stabiliser cette émulsion. Mais, les alcanols de C1 à C4 présentent l'inconvénient d'être irritants sur la peau ou les muqueuses et donc de contribuer à l'inconfort décrit ci-dessus des hydroxyacides.

Le but de la présente invention est donc de proposer une composition à teneur élevée en hydroxyacide stable dans le temps.

Un autre but de l'invention est de proposer une composition comprenant des hydroxyacides qui ne présente pas l'inconfort, tel que décrit ci-dessus, lorsque la composition est appliquée sur la peau ou les muqueuses.

Ainsi, ces buts et d'autres sont atteints par la présente invention qui concerne une composition cosmétique ou dermatologique du type eau dans huile exempte d'alcanol en C₁ à C₄, caractérisée en ce qu'elle comprend :
- de 10 à 30 % en poids d'hydroxyacide;
- de 1 à 15 % en poids d'au moins une silicone à substitution polyoxyalkylène de formule générale (I): 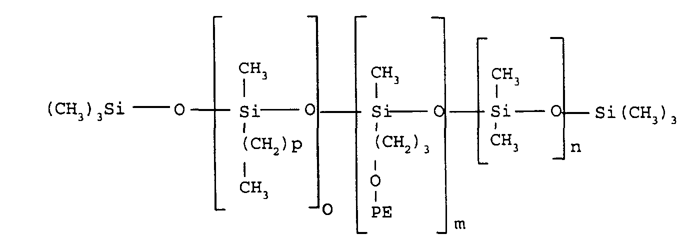 formule dans laquelle :
   - PE représente (-C₂H₄O)ₓ(-C₃H₆O)_{y}-R, R étant choisi parmi un atome d'hydrogène et un radical alkyle de 1 à 4 atomes de carbone, x étant compris entre 10 et 100 et y compris entre 0 et 80;
   - m est compris entre 1 et 25;
   - n est compris entre 10 et 200;
   - o est compris entre 1 et 100;
   - p est compris entre 7 et 17;
   sous réserve que o n'est pas inférieur à m et que 3o est inférieur à n, et dans laquelle le poids moléculaire de PE est compris entre 250 et 2000 avec x et y choisis de manière à ce que leur rapport en poids x/y soit compris entre 100/0 et 20/80; et
- de 0,1 à 6 % en poids d'au moins un composé co-émulsionnant choisi parmi un ester alkylé de polyol, un éther alkylé de polyol et un éther alkylé à substitution oxyalkylène.

Malgré la quantité importante d'hydroxyacide, la composition présente une stabilité au stockage. Ainsi, la composition selon l'invention peut être stable à une température de 45°C pendant au moins trois mois.

De plus, grâce à cette formulation spécifique, la libération de l'actif hydroxyacide est réalisée de manière lente et différée lorsque cette composition est appliquée sur la peau, les ongles, les cheveux et/ou le cuir chevelu, ce qui rend celle-ci très agréable pour l'utilisateur.

Les hydroxyacides auxquels s'applique l'invention peuvent être des α-hydroxy-acides ou des β-hydroxy-acides, qui peuvent être linéaires, ramifiés ou cycliques, saturés ou insaturés. Les atomes d'hydrogène de la chaîne carbonée peuvent, en outre, être substitués par des halogènes, des radicaux halogénés, alkylés, acylés, acyloxylés, alcoxy carbonylés ou alcoxylés ayant de 2 à 18 atomes de carbone. On peut également utiliser les α- et β-cétoacides.

Les hydroxy-acides les plus utilisés en cosmétique ou en dermatologie sont les acides glycolique, lactique, malique, tartrique, citrique, mandélique, salicylique ainsi que leurs dérivés alkylés comme l'acide n-octanoyl-5-salicylique, l'acide n-dodécanoyl-5-salicylique, l'acide 2-hydroxy-3-méthylbenzoïque, ou encore leurs dérivés alcoxylés comme l'acide 2-hydroxy-3-méthoxybenzoïque. On utilise préférentiellement selon l'invention l'acide lactique, l'acide glycolique ou l'acide citrique.

Cet α- ou β-hydroxyacide peut bien entendu consister en un acide mono ou poly carboxylique comportant une ou plusieurs fonctions hydroxy, l'une au moins de ces fonctions hydroxy devant occuper une position α ou β sur ledit acide.

Les compositions conformes à l'invention peuvent bien entendu contenir un ou plusieurs hydroxyacides.

Dans tout ce qui suit ou ce qui précède, la quantité en hydroxyacide utilisé est exprimée en pourcentage d'acide libre, sauf mention contraire.

De préférence, la quantité en hydroxyacide utilisé est comprise entre 12 et 28 % en poids par rapport au poids total de la composition.

La silicone à substitution polyoxyalkylène est plus particulièrement utilisée dans la présente invention pour son pouvoir émulsionnant.

On utilise avantageusement une silicone à substitution polyoxyalkylène répondant à la formule générale (I) dans laquelle m égal à 5, n égal à 75, o compris entre 20 et 25, p égal à 15, PE est complètement polyoxyéthyléné et a un poids moléculaire de 400. Ainsi, on utilise plus particulièrement le cétyl diméthicone copolyol vendu sous la dénomination commerciale Abil EM 90 ® par la société Goldschmidt.

La composition selon l'invention comprend préférentiellement de 1 à 5 % en poids d'au moins une silicone à substitution polyoxyalkylène.

La chaîne alkyle du composé co-émulsionnant utilisé dans la présente invention présente de préférence de 12 à 20 atomes de carbone.

Le co-émulsionnant éther alkylé à substitution oxyalkylène présente généralement de 2 à 10 motifs oxyalkylène par molécule d'éther.

Le co-émulsionnant éther alkylé à substitution oxyalkylène est avantageusement un éther gras à substitution oxyalkylène, généralement oxyéthylène et/ou oxypropylène. Il présente avantageusement un nombre de motifs oxyalkylène compris entre 2 et 4 par molécule d'éther.

Parmi les éthers gras à substitution oxyalkylène utilisés dans la présente invention, on préfère l'éther d'alcool myristique et de polypropylène glycol, tel que le Witconol APM ® (3 moles d'oxyde de propylène) vendu par la société Witco.

Le co-émulsionnant ester alkylé de polyol ou éther alkylé de polyol est avantageusement un ester gras ou un éther gras polyglycérolé, portant généralement de 2 à 10 motifs glycérol par molécule d'ester ou d'éther, ou un ester gras ou un éther gras dérivé de sucre, tel que sorbitol, sorbitanne ou méthylglucoside, ou encore un ester gras ou un éther gras de glycérol et de sucre, tel que sorbitol, sorbitanne ou méthylglucoside.

Les esters alkylés de polyol ou éthers alkylés de polyol peuvent être des mono-, di- ou polyesters ou éthers.

Ces co-émulsionnants peuvent être utilisés seuls ou en mélange.

Parmi les esters alkylés de polyol ou éthers alkylés de polyol, on préfére l'isostéarate de polyglycérol, tel que l'Isolan GI 34 ® vendu par la société Goldschmidt, l'isostéarate de sorbitanne, tel que l'Arlacel 987 ® vendu par la société ICI, l'isostéarate de sorbitanne et de glycérol, tel que l'Arlacel 986 ® vendu par la société ICI ou le sesquistéarate de méthylglucose, tel que le glycate IS ® vendu par la société Amerchol.

La quantité de composé co-émulsionnant choisi parmi un ester alkylé de polyol, un éther alkylé de polyol et un éther alkylé à substitution oxyalkyléne dans la composition est avantageusement comprise entre 0,1 et 3 % en poids.

Parmi les alcanols de C1 à C4 non compris dans la composition selon l'invention, on peut citer l'éthanol, le n-propanol, l'isopropanol et le n-butanol.

La phase grasse de la composition peut comprendre des dérivés hydrocarbonés saturés ou insaturés, tels que l'huile de vaseline ou le perhydrosqualène, ou des esters gras aliphatiques ou aromatiques, tels que le benzoate alkylé de C₁₂-C₁₅, le dodécanol octylé ou le stéarate octylé, ou des triglycérides, tels que les triglycérides d'acide caprique ou caprylique, ou encore des polydiméthylsiloxanes.

La proportion de la phase grasse varie avantageusement de 20 à 40 % du poids total de la composition selon l'invention.

La phase grasse de la composition comprend préférentiellement au moins un polydiméthylsiloxane, leur quantité dans la composition étant généralement comprise entre 10 et 30 % en poids.

On peut ainsi utiliser des polydiméthylcyclosiloxanes volatiles ayant une viscosité de moins de 5 mm²s⁻¹, tel que notamment les cyclométhicone tétramère ou pentamère de Dow Corning (Dow Corning 344 Fluid et Dow Corning 345 Fluid) ou encore l'hexaméthyldisiloxane volatile ayant une viscosité de moins de 0,65 mm²s⁻¹, tel que notamment le Rhodorsil Oils de Rhône-Poulenc (RP 70041VO65). Il est possible d'utiliser, également, au moins un polydiméthylsiloxane non volatile tel que le polydiméthylsiloxane ayant une viscosté supérieure à 5 mm²s⁻¹, notamment comprise entre 50 et 1000 mm²s⁻¹, tels que par exemple Dow Corning 200 Fluid.

On peut les utiliser seuls ou de préférence en mélange.

Pour augmenter éventuellement la stabilité de la composition à basses températures ou pour éviter éventuellement la recristallisation de certains hydroxyacides ou encore pour éventuellement rendre la composition transparente, on peut ajouter à la composition des polyols, tels que le glycérol, le 1,2 propylène glycol et le sorbitol, dans des quantités généralement comprises entre 0,5 et 15 % en poids.

Pour augmenter éventuellement la stabilité de la composition à des températures plus élevées, on peut ajouter à la composition des cires naturelles ou synthétiques, telles que la cire d'abeille, la cire de Carnauba, le beurre de Karité, ou des cires de silicone ou des dispersions lipidiques d'hectorites modifiées ou encore du stéarate d'aluminium ou de calcium ou des électrolytes, tels que du chlorure de sodium, du sulfate de magnésium.

La composition selon l'invention peut également comprendre des microparticules de dioxyde de titane présentant un diamètre moyen compris entre 1 et 100 nm, de préférence, de 10 à 40 nm. Deux formes de dioxyde de titane sont disponibles, un type dispersible dans la phase aqueuse et un autre type dispersible dans la phase grasse.

Les particules de dioxyde de titane du type dispersible dans la phase aqueuse peuvent être des particules non enrobées ou des particules enrobées avec un matériel leur conférant une surface hydrophile, tel que l'oxyde d'aluminium ou le silicate d'aluminium.

Les particules de dioxyde de titane du type dispersible dans la phase grasse sont des particules enrobées avec un matériel leur conférant une surface lipophile, tel que le stéarate d'aluminium, le laurate d'aluminium, le stéarate de zinc, ou des composés organiques, éventuellement siliciés.

La quantité de dioxyde de titane dans la composition selon la présente invention est généralement comprise entre 0,5 et 2 % en poids.

La composition selon l'invention peut, bien entendu, en outre comprendre des additifs inertes ou même cosmétiquement ou dermatologiquement actifs ou des combinaisons de ces additifs.

On peut envisager d'ajouter des agents d'amélioration de la saveur, des agents conservateurs, tels que les esters de l'acides parahydroxybenzoïque, les agents stabilisants, des agents régulateurs d'humidité, des agents régulateurs du pH.

La composition selon l'invention peut donc contenir en outre un agent actif destiné notamment au traitement ou à la prévention des affections cutanées, y compris l'acné, les mycoses, les dermites séborrhéiques, l'eczéma, la rosacée, les héliodermatoses et le vieillissement cutané, ou encore les affections du cuir chevelu ou des ongles.

Parmi les agents dermatologiquement actifs, on peut citer à titre d'exemple :
- les agents modulant la différenciation et/ou la prolifération et/ou la pigmentation cutanée tels que les composés dont l'action est médiée par les récepteurs nucléaires de la super-famille des stéroïdes/hormones thyroïdiennes, en particulier l'acide rétinoïque, ses isomères et ses dérivés par exemple le rétinol ou le rétinaldéhyde, ainsi que des composés de synthèse analogues ; la vitamine D ou ses dérivés ; les estrogènes ; les antinéoplasiques, tels que le 5-fluorouracyle ;
- les agents antibiotiques, tels que le phosphate de clindamycine, l'érythromycine ou les antibiotiques de la classe des tétracyclines ;
- les antibactériens, en particulier le peroxyde de benzoyle ;
- les antiinfectieux, en particulier le métronidazole ;
- les antifongiques, en particulier les composés appartenant à la famille des imidazoles, les composés polyènes, tels que l'amphotéricine B ou les composés de la famille des allylamines, tels que la terbinafine ;
- les agents anti-inflammatoires stéroïdiens, tels que l'hydrocortisone, le valérate de bétaméthasone ou le propionate de clobétasol, ou les agents anti-inflammatoires non-stéroïdiens tels que l'ibuprofène ou le diclofénac ou leurs sels ;
- les agents anti-prurigineux, tels que la capsaïcine ou les inhibiteurs de NK1 ou les sels de lithium ;
- les agents analgésiques ;
- les agents anti-viraux, tels que l'acyclovir ;
- les bloqueurs de canaux ioniques, tels que le minoxidil et ses dérivés ;
- les anti-radicaux libres, tels que l'alpha-tocophérol ou ses esters, les superoxyde dismutases ou certains chélatants des métaux.

La concentration utilisée dans la composition dépend bien entendu de la nature de l'agent actif et du résultat attendu, elle est généralement comprise entre 0,001% et 10% en poids de la composition.

De manière avantageuse, la composition selon l'invention ne comprend pas de composés de natures ou en quantités telles qu'ils rendent la composition inconfortable lorsqu'elle est appliquée sur la peau ou les muqueuses.

La composition selon l'invention se présente généralement sous forme de lait, de crème ou d'une émulsion transparente.

La composition selon l'invention peut être préparée de manière connue en soi. Ainsi, il est possible de dissoudre ou de disperser l'émulsionnant ou le mélange émulsionnant dans la phase grasse. L'eau est ensuite ajoutée, de préférence lentement. L'agitation peut être réalisée par tout moyen connu en soi. On préfére agiter de façon à former une pré-émulsion. Les autres composés de l'émulsion peuvent être simplement ajoutés au préalable à la phase dans laquelle ils sont solubles ou dispersibles. L'émulsion peut être stabilisée avec un système d'agitation, du type rotor-stator, de préférence à vitesse élevée.

La composition selon l'invention est plus particulièrement destinée, en application topique, au traitement ou au soin de la peau, des ongles, des cheveux et/ou du cuir chevelu, et de préférence pour traiter et/ou prévenir la xérose, l'ichtyose, la kératose actinique et/ou le vieillissement cutané photoinduit.

Dans tout ce qui suit et ce qui précède, les pourcentages sont donnés en poids sauf mention contraire.

On va maintenant donner, à titre d'illustration et sans aucun caractère limitatif, plusieurs exemples de préparation des compositions selon l'invention.

### EXEMPLES

### Procédé général de formulation des exemples suivants

On dissout ou on disperse l'émulsionnant ou le mélange émulsionnant dans la phase grasse. L'eau est ensuite ajoutée lentement. L'agitation est moyenne de façon à former une pré-émulsion. Les autres composés de l'émulsion sont ajoutés au préalable à la phase dans laquelle ils sont solubles ou dispersibles. L'émulsion est stabilisée avec un système d'agitation, du type rotor-stator et ceci à vitesse élevée.

### Dans ces exemples :

POB signifie parahydroxybenzoate ;
PPG signifie polypropylène glycol ;
POP signifie polyoxypropylène ;
POE signifie polyoxyéthylène ;

### EXEMPLE 1

| Formulation | | |
|---|---|---|
| Nom commercial | Nom chimique | % |
| *PHASE GRASSE* | | |
| Abil EM 90® (Goldschmidt) | Cetyl dimethicone copolyol | 2.50 |
| Witconol APM® | PPG-3 myristyl ether | 0.50 |
| Dow Corning 344 fluid® | Cyclométhicone | 9.00 |
| Dow Corning 345 fluid® | Cyclométhicone | 9.00 |
| Dow Corning 200 fluid® | Diméthicone | 2.00 |
| TiO2 PW Covasil S® | TiO2+alkyl silane+polyméthylméthacrylate | 1.00 |

| *PHASE AQUEUSE* | | |
|---|---|---|
| Glycérol | | 3.00 |
| Sorbitol 70% | | 5.00 |
| Acide citrique, 1H2O | | 27.30 (25 % anhydre) |
| POB méthyle | | 0.15 |
| NaCI | | 0.60 |
| Ammoniaque 32% | QS | pH 3(#) |
| Eau | QS | 100 |

| | | |
|---|---|---|
| (#) soit 4.50% d'ammoniaque à 32% | | |

Cette composition présente l'avantage d'être stable et d'être bien tolérée par la peau humaine, malgré la grande quantité d'acide citrique présent (=25%).

### EXEMPLE 2

| Formulation | | |
|---|---|---|
| Nom commercial | Nom chimique | % |
| *PHASE GRASSE* | | |
| Abil EM 90® (Goldschmidt) | Cetyl dimethicone copolyol | 2.50 |
| Witconol APM® | PPG-3 myristyl ether | 0.50 |
| Dow Corning 344 fluid® | Cyclométhicone | 9.00 |
| Dow Corning 345 fluid® | Cyclométhicone | 9.00 |
| Dow Corning 200 fluid® | Diméthicone | 2.00 |
| TiO2 PW Covasil S® | TiO2+alkyl silane+polyméthylméthacrylate | 1.00 |

| *PHASE AQUEUSE* | | |
|---|---|---|
| Acide citrique, 1H2O | | 27.30 (25 % anhydre) |
| POB méthyle | | 0.15 |
| NaCI | | 0.60 |
| Ammoniaque 32% | QS | pH 3(#) |
| Eau | QS | 100 |

| | | |
|---|---|---|
| (#) soit 4.50% d'ammoniaque à 32% | | |

Cette composition est stable pendant 6 mois à température ambiante.

### EXEMPLE COMPARATIF 3

| Formulation | | |
|---|---|---|
| Nom commercial | Nom chimique | % |
| Arlacel 780® (ICI) | POP-POE glycérol sorbitanne hydroxyisostéarate | 6 |
| Vaseline épaisse | | 10 |
| Dow Corning 344 Fluid | cyclométhicone | 15 |
| Eau | | 50,18 |
| Piperazine | | 4,82 |
| Acide lactique (90%) | | 12,00 |
| Chlorure de sodium | | 2,00 |

Après un mois à température de 45°C, la composition présente un exsudat important.

### EXEMPLE COMPARATIF 4

| Formulation | | |
|---|---|---|
| Nom commercial | Nom chimique | % |
| Arlacel 582® (ICI) | POE glycérol sorbitanne isostéarate | 1 |
| Arlatone T® (ICI) | POE(40)-sorbitolseptaoléate | 4 |
| Huile vaseline fluide | huile minérale | 10 |
| Rilanit G16S® (Henkel) | isocétyle stéarate | 10 |
| POB propyle | | 0,05 |
| eau | | 47,95 |
| POB méthyle | | 0,10 |
| Glycérine | glycérol | 3 |
| Lubragel CG® (Sederma) | glycéryl polyméthacrylate et propylène glycol | 5 |
| Acide glycolique | | 12 |
| Hydroxyde de sodium | | 4,68 |
| Chlorure de sodium | | 2,00 |

Après un mois à température de 45°C, la composition présente un exsudat important.

### EXEMPLE COMPARATIF 5

| Formulation | | |
|---|---|---|
| Nom commercial | Nom chimique | % |
| Abil EM 90® (Goldschmidt) | cétyl diméthicone copolyol | 2,5 |
| Eutanol G® (Henkel) | octyldodécanol | 9,5 |
| Dow Corning 344 Fluid® | cyclométhicone | 15 |
| eau | | 34,75 |
| POB méthyle | | 0,15 |
| Glycérine | glycérol | 3,00 |
| Lubragel CG® (Sederma) | glycéryl polyméthacrylate et propylène glycol | 5,00 |
| Acide citrique anhydre | | 25,00 |
| Ammoniaque à 32% | | 4,50 |
| Chlorure de sodium | | 0,60 |

Cette composition, sans composé co-émulsionnant, exsude dès le premier mois de stabilité.

### EXEMPLE COMPARATIF 6

| Formulation | | |
|---|---|---|
| Nom commercial | Nom chimique | % |
| Arlacel 60® (ICI) | sorbitanne monostéarate | 4,5 |
| Amphisol K® (Givaudan) | potassium cétyl phosphate | 0,50 |
| Rilanit G16S® (Henkel) | isocétyl stéarate | 4,00 |
| Alcool stéarylique | | 4,00 |
| Dow Corning 200® (350 cps) | diméthicone | 0,50 |
| POB propyle | | 0,05 |
| Huile de vaseline fluide | huile minérale fluide | 3,00 |
| Eau | | 39,25 |
| POB méthyle | | 0,10 |
| Glycérine | glycérol | 3,00 |
| Phénoxyéthanol | | 0,50 |
| Lubragel CG® (Sederma) | glycéryl polyméthacrylate et propylène glycol | 5,00 |
| Acide citrique anhydre | | 25,00 |
| Ammoniaque à 32% | | 8,80 |
| Methocel E4M Premium® (Dow Chemical) | hydroxypropylméthylcellulose | 0,30 |
| Veegum HS® (Vanderbilt) | magnésium aluminium silicate | 1,50 |

Cette composition de type émulsion huile dans eau présente un déphasage dès le premier jour de stabilité.

## Revendications

1. Composition cosmétique ou dermatologique du type eau-dans-l'huile exempte d'alcanol en C₁ à C₄, **caractérisée en ce qu'**elle comprend :
- de 10 à 30 % en poids d'hydroxyacide,
- de 1 à 15 % en poids d'au moins une silicone à substitution polyoxyalkylène de formule générale (I): 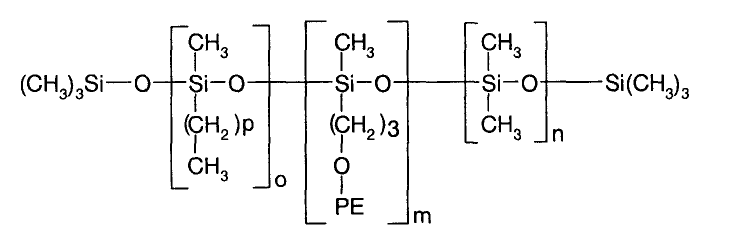 dans laquelle:
PE représente (-C₂H₄O)ₓ(-C₃H₆O)_{y}-R, R étant choisi parmi un atome d'hydrogène et un radical alkyle de 1 à 4 atomes de carbone,
m est compris entre 1 et 25,
n est compris entre 10 et 200,
o est compris entre 1 et 100,
p est compris entre 7 et 17,
sous réserve que o n'est pas inférieur à m et que 3o est inférieur à n, formule dans laquelle le poids moléculaire de PE est compris entre 250 et 2000 avec x et y choisis de manière à ce que le rapport en poids x/y soit compris entre 100/0 et 20/80, et
- de 0,1 à 6 % en poids d'au moins un composé co-émulsionnant choisi parmi un ester alkylé de polyol, un éther alkylé de polyol et un éther alkylé à substitution oxyalkylène.

2. Composition selon la revendication 1, **caractérisée en ce que** l'hydroxyacide est choisi parmi l'acide glycolique, lactique, malique, tartrique, citrique, mandélique, salicylique, n-octanoyl-5-salicylique, n-docécanoyl-5-salicylique, 2-hydroxy-3-méthylbenzoïque et l'acide 2-hydroxy-3-méthoxybenzoïque.

3. Composition selon la revendication 2, **caractérisée en ce que** l'hydroxyacide est choisi parmi l'acide lactique, l'acide glycolique et l'acide citrique.

4. Composition selon l'une quelconque des revendications précédentes, **caractérisée en ce que** la quantité d'hydroxyacide utilisée est comprise entre 12 et 28 % en poids par rapport au poids total de la composition.

5. Composition selon l'une quelconque des revendications précédentes, **caractérisée en ce qu'**elle comprend de 1 à 5 % en poids d'au moins un silicone à substitution polyoxyalkylène.

6. Composition selon l'une quelconque des revendications précédentes, **caractérisée en ce que** la chaîne alkyle du composé co-émulsionnant utilisé contient de 12 à 20 atomes de carbone.

7. Composition selon l'une quelconque des revendications précédentes, **caractérisée en ce que** la quantité du composé co-émulsionnant est comprise entre 0,1 et 3 % en poids.

8. Composition selon l'une quelconque des revendications précédentes, **caractérisée en ce que** l'éther alkylé à substitution oxyalkylène, du type oxyéthylène et/ou oxypropylène, présente un nombre de motifs oxyalkylène compris entre 2 et 10, de préférence entre 2 et 4, par molécule d'éther.

9. Composition selon l'une quelconque des revendications 1 à 8, **caractérisée en ce que** l'ester alkylé de polyol ou l'éther alkylé de polyol est un ester gras ou un éther gras polyglycérolé, portant de préférence de 2 à 10 motifs de glycérol, ou un ester gras ou un éther gras dérivé de sucre, tel que sorbitol, sorbitanne ou méthylglucoside, ou encore un ester gras ou un éther gras de glycérol et de sucre, tel que sorbitol, sorbitanne ou méthylglucoside.

10. Composition selon la revendication 9, **caractérisée en ce que** l'ester alkylé de polyol ou l'éther alkylé de polyol est choisi parmi l'isostéarate de polyglycérol, l'isostéarate de sorbitanne, l'isostéarate de sorbitanne et de glycérol et le sesquistéarate de méthylglucose.

11. Composition selon l'une quelconque des revendications précédentes, **caractérisée en ce que** la phase grasse de la composition comprend des dérivés hydrocarbonés saturés ou insaturés, tels que l'huile de vaseline ou le perhydrosqualène, ou des esters gras aliphatiques ou aromatiques, tels que le benzoate alkylé en C₁₂-C₁₅, le dodécanol octylé ou le stéarate octylé, ou des triglycérides, tels que les triglycérides d'acide caprique ou caprylique, ou encore des polydiméthylsiloxanes.

12. Composition selon l'une quelconque des revendications précédentes, **caractérisée en ce que** la proportion de la phase grasse varie de 20 à 40 % du poids total de la composition selon l'invention.

13. Composition selon l'une quelconque des revendications précédentes, **caractérisée en ce que** la phase grasse de la composition comprend au moins un polydiméthylsiloxane.

14. Composition selon l'une quelconque des revendications précédentes, **caractérisée en ce qu'**elle comprend des polyols, tels que le glycérol, le 1,2-propylène glycol et le sorbitol, dans des quantités comprises entre 0,5 et 15 % en poids.

15. Composition selon l'une quelconque des revendications précédentes, **caractérisée en ce qu'**elle comprend des cires naturelles ou synthétiques, telles que la cire d'abeille, la cire de Carnauba, le beurre de Karité, ou des cires de silicone ou des dispersions lipidiques d'hectorites modifiées ou encore du stéarate d'aluminium ou de calcium ou des électrolytes, tels que du chlorure de sodium ou du sulfate de magnésium.

16. Composition selon l'une quelconque des revendications précédentes, **caractérisée en ce qu'**elle comprend des microparticules de dioxyde de titane présentant un diamètre compris entre 1 et 100 nm, de préférence, de 10 à 40 nm.

17. Composition selon la revendication 16, **caractérisée en ce que** la quantité de dioxyde de titane est comprise entre 0,5 et 2 % en poids.

18. Composition selon l'une quelconque des revendications précédentes, **caractérisée en ce qu'**elle est destinée, en application topique, au traitement ou au soin de la peau, des ongles, des cheveux et/ou du cuir chevelu, et de préférence pour traiter et/ou prévenir la xérose, l'ichtyose, la kératose actinique et/ou le vieillissement cutané photoinduit.

## Patentansprüche

1. Kosmetische oder dermatologische Zusammensetzung vom Typ einer Wasser-in-Öl-Emulsion, die kein C₁₋₄-Alkanol enthält, **dadurch gekennzeichnet, daß** sie enthält:
- 10 bis 30 Gew.-% Hydroxysäure,
- 1 bis 15 Gew.-% mindestens eines polyoxyalkylensubstituierten Silicons der allgemeinen Formel (I), 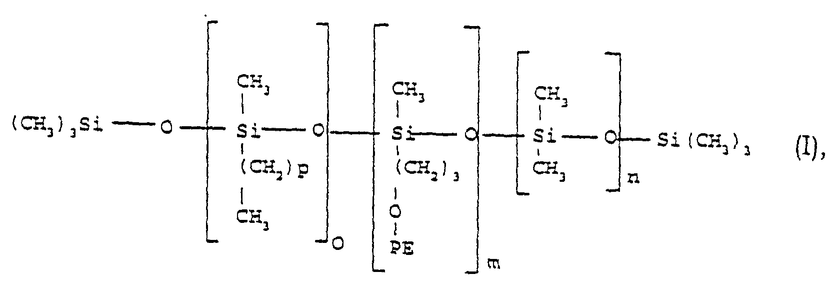 in der bedeuten:
- PE (-C₂H₄O)ₓ(-C₃H₆O)_{y}-R, wobei R unter Wasserstoff und C₁₋₄-Alkyl ausgewählt ist und x im Bereich von 10 bis 100 und y im Bereich von 0 bis 80 liegen,
- m eine Zahl von 1 bis 25,
- n eine Zahl von 10 bis 200,
- o eine Zahl von 1 bis 100 und
- p eine Zahl von 7 bis 17,
mit der Maßgabe, daß o nicht kleiner als m ist und 3o kleiner als n ist, und bei der das Molekulargewicht von PE im Bereich von 250 bis 2000 liegt und x und y so ausgewählt sind, daß das Gewichtsverhältnis x/y im Bereich von 100/0 bis 20/80 liegt,
und
- 0,1 bis 6 Gew.-% mindestens eines Coemulgators, der ausgewählt ist unter Polyolalkylestern, Polyolalkylethern und oxyalkylensubstituierten Alkylethern.

2. Zusammensetzung nach Anspruch 1, **dadurch gekennzeichnet, daß** die Hydroxysäure ausgewählt ist unter Glykolsäure, Milchsäure, Äpfelsäure, Weinsäure, Citronensäure, Mandelsäure und Salicylsäure sowie ihren Alkylderivaten, wie 5-n-Octanoylsalicylsäure, 5-n-Dodecanoylsalicylsäure und 2-Hydroxy-3-methylbenzoesäure, sowie ihren Alkoxyderivaten, wie 2-Hydroxy-3-methoxybenzoesäure.

3. Zusammensetzung nach Anspruch 2, **dadurch gekennzeichnet, daß** die Hydroxysäure unter Milchsäure, Glykolsäure und Citronensäure ausgewählt ist.

4. Zusammensetzung nach einem der vorhergehenden Ansprüche, **dadurch gekennzeichnet, daß** die Menge an eingesetzter Hydroxysäure 12 bis 28 Gew.-%, bezogen auf das Gesamtgewicht der Zusammensetzung, beträgt.

5. Zusammensetzung nach einem der vorhergehenden Ansprüche, **dadurch gekennzeichnet, daß** sie 1 bis 5 Gew.-% mindestens eines polyoxyalkylensubstituierten Silicons enthält.

6. Zusammensetzung nach einem der vorhergehenden Ansprüche, **dadurch gekennzeichnet, daß** die Alkylkette des eingesetzten Coemulgators 12 bis 20 C-Atome aufweist.

7. Zusammensetzung nach einem der vorhergehenden Ansprüche, **dadurch gekennzeichnet, daß** die Menge des Coemulgators im Bereich von 0,1 bis 3 Gew.-% liegt.

8. Zusammensetzung nach einem der vorhergehenden Ansprüche, **dadurch gekennzeichnet, daß** der oxyalkylensubstituierte Alkylether vom mit Oxyethylengruppen und/oder Oxypropylengruppen substituierten Typ ist, pro Ethermolekül eine Anzahl von Oxyalkylengruppen aufweist, die im Bereich von 2 bis 10 und vorzugsweise im Bereich von 2 bis 4 liegt.

9. Zusammensetzung nach einem der Ansprüche 1 bis 8, **dadurch gekennzeichnet, daß** der Polyolalkylester oder Polyolalkylether ein Polyglycerinfettester oder ein Polyglycerinfettether mit vorzugsweise 2 bis 10 Glycerineinheiten oder ein von einem Zukker, wie Sorbit, Sorbitan oder Methylglucosid, abgeleiteter Fettester oder Fettether oder ein von Glycerin und einem Zucker, wie Sorbit, Sorbitan oder Methylglucosid, abgeleiteter Fettester oder Fettether ist.

10. Zusammensetzung nach Anspruch 9, **dadurch gekennzeichnet, daß** der Polyolalkylester oder der Polyolalkylether unter Polyglycerinisostearat, Sorbitanisostearat, Sorbitan- und Glycerinisostearat sowie Methylglucosesesquistearat ausgewählt ist.

11. Zusammensetzung nach einem der vorhergehenden Ansprüche, **dadurch gekennzeichnet, daß** die Fettphase der Zusammensetzung gesättigte oder ungesättigte Kohlenwasserstoffderivate, wie Vaselinöl oder Perhydrosqualen, aliphatische oder aromatische Fettester, wie C₁₂₋₁₅-Alkylbenzoate, Octyldodecanol oder Octylstearat, oder Triglyceride, wie Caprinsäure- oder Caprylsäure-Triglyceride, oder auch Polydimethylsiloxane enthält.

12. Zusammensetzung nach einem der vorhergehenden Ansprüche, **dadurch gekennzeichnet, daß** der Mengenanteil der Fettphase 20 bis 40 Gew.-%, bezogen auf das Gesamtgewicht der erfindungsgemäßen Zusammensetzung, beträgt.

13. Zusammensetzung nach einem der vorhergehenden Ansprüche, **dadurch gekennzeichnet, daß** die Fettphase der Zusammensetzung mindestens ein Polydimethylsiloxan enthält.

14. Zusammensetzung nach einem der vorhergehenden Ansprüche, **dadurch gekennzeichnet, daß** sie Polyole, wie Glycerin, 1,2-Propylenglykol und Sorbit, in Mengenanteilen von 0,5 bis 15 Gew.-% enthält.

15. Zusammensetzung nach einem der vorhergehenden Ansprüche, **dadurch gekennzeichnet, daß** sie natürliche oder synthetische Wachse, wie Bienenwachs, Carnaubawachs oder Shibutter, Siliconwachse, Lipiddispersionen von modifizierten Hectoriten, Aluminiumstearat, Calciumstearat oder Elektrolyte, wie Natriumchlorid oder Magnesiumsulfat, enthält.

16. Zusammensetzung nach einem der vorhergehenden Ansprüche, **dadurch gekennzeichnet, daß** sie Titandioxid-Mikropartikel einer mittleren Teilchengröße von 1 bis 100 nm und vorzugsweise 10 bis 40 nm enthält.

17. Zusammensetzung nach Anspruch 16, **dadurch gekennzeichnet, daß** der Mengenanteil an Titandioxid 0,5 bis 2 Gew.-% beträgt.

18. Zusammensetzung nach einem der vorhergehenden Ansprüche, **dadurch gekennzeichnet, daß** sie zur Behandlung oder zur Pflege der Haut, der Nägel, der Haare und/oder der Kopfhaut in topischer Anwendung vorgesehen ist, vorzugsweise zur Behandlung und/oder Vorbeugung von Xerose, Ichthyose, Strahlenkeratose und/oder lichtinduzierter Hautalterung.

## Claims

1. Cosmetic or dermatological composition of the water-in-oil type which is devoid of C₁ to C₄ alkanol, **characterized in that** it includes:
- from 10 to 30% by weight of hydroxy acid;
- from 1 to 15% by weight of at least one silicone with polyoxyalkylene substitution of general formula (I) : 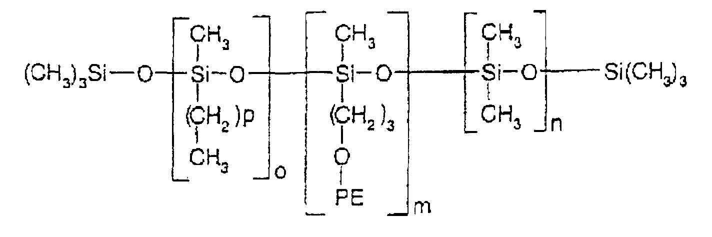 in which:
- PE denotes (-C₂H₄O)ₓ(-C₃H₆O)_{y}-R, R being chosen from a hydrogen atom and an alkyl radical containing 1 to 4 carbon atoms,
m is between 1 and 25;
n is between 10 and 200;
o is between 1 and 100;
p is between 7 and 17;
with the proviso that o is not smaller than m and that 3o is smaller than n, in which formula molecular weight of PE is between 250 and 2000 with x and y chosen so that their weight ratio x/y is between 100/0 and 20/80, and
- from 0.1 to 6% by weight of at least one coemulsifying compound chosen from a polyol alkyl ester, a polyol alkyl ether and an alkyl ether with oxyalkylene substitution.

2. Composition according to Claim 1, **characterized in that** the hydroxy acid is chosen from glycolic, lactic, malic, tartaric, citric, mandelic, salicylic, 5-n-octanoylsalicylic, 5-n-dodecanoylsalicylic, 2-hydroxy-3-methylbenzoic and 2-hydroxy-3-methoxybenzoic acid.

3. Composition according to Claim 2, **characterized in that** the hydroxy acid is chosen from lactic acid, glycolic acid or citric acid.

4. Composition according to any one of the preceding claims, **characterized in that** the quantity of hydroxy acid employed is between 12 and 28% by weight relative to the total weight of the composition.

5. Composition according to any one of the preceding claims, **characterized in that** it includes from 1 to 5% by weight of at least one silicone with polyoxyalkylene substitution.

6. Composition according to any one of the preceding claims, **characterized in that** the alkyl chain of the coemulsifying compound employed has from 12 to 20 carbon atoms.

7. Composition according to any one of the preceding claims, **characterized in that** the quantity of coemulsifying compound is between 0.1 and 3% by weight.

8. Composition according to any one of the preceding claims, **characterized in that** the alkyl ether with oxyalkylene substitution, of the oxyethylene and/or oxypropylene type, has a number of oxyalkylene units which is between 2 and 10, preferably between 2 and 4, per molecule of ether.

9. Composition according to any one of Claims 1 to 8, **characterized in that** the polyol alkyl ester or the polyol alkyl ether is a polyglycerolated fatty ester or fatty ether, preferably containing from 2 to 10 glycerol units, or a fatty ester or a fatty ether derived from a sugar such as sorbitol, sorbitan or methyglucoside, or else a fatty ester or a fatty ether of glycerol and of a sugar such as sorbitol, sorbitan or methylglucoside.

10. Composition according to Claim 9, **characterized in that** the polyol alkyl ester or the polyol alkyl ether is chosen from polyglycerol isostearate, sorbitan isostearate, sorbitan glycerol isostearate and methylglucose sesquistearate.

11. Composition according to any one of the preceding claims, **characterized in that** the fatty phase of the composition includes saturated or unsaturated hydrocarbon derivatives such as liquid petrolatum or perhydrosqualene or aliphatic or aromatic fatty esters such as a C₁₂-C₁₅ alkyl benzoate, octyldodecanol or octyl stearate or triglycerides such as the triglycerides of capric or caprylic acid or else polydimethylsiloxanes.

12. Composition according to any one of the preceding claims, **characterized in that** the proportion of the fatty phase varies from 20 to 40% of the total weight of the composition according to the invention.

13. Composition according to any one of the preceding claims, **characterized in that** the fatty phase of the composition includes at least one polydimethylsiloxane.

14. Composition according to any one of the preceding claims, **characterized in that** it includes polyols such as glycerol, 1,2-propylene glycol and sorbitol, in quantities of between 0.5 and 15% by weight.

15. Composition according to any one of the preceding claims, **characterized in that** it includes natural or synthetic waxes such as beeswax, carnauba wax, butter tree butter or silicone waxes or lipid dispersions of modified hectorites or else aluminium or calcium stearate or electrolytes such as sodium chloride or magnesium sulphate.

16. Composition according to any one of the preceding claims, **characterized in that** it includes titanium dioxide microparticles which have a mean diameter of between 1 and 100 nm, preferably from 10 to 40 nm.

17. Composition according to Claim 16, **characterized in that** the quantity of titanium dioxide is between 0.5 and 2% by weight.

18. Composition according to any one of the preceding claims **characterized in that** it is intended, in topical application, for the treatment or care of the skin, nails, hair and/or scalp, and preferably for treating and/or preventing xerosis, ichthyosis, actinic keratosis and/or photoinduced cutaneous aging.
